(19) Europäisches Patentamt
European Patent Office
Office européen des brevets

(11) **EP 4 653 046 A1**

(12) **EUROPEAN PATENT APPLICATION**

(43) Date of publication:
**26.11.2025 Bulletin 2025/48**

(21) Application number: 25178182.9

(22) Date of filing: **22.05.2025**

(51) International Patent Classification (IPC):
***A61N 5/10*** *(2006.01)*

(52) Cooperative Patent Classification (CPC):
**A61N 5/103;** A61N 2005/1087

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **24.05.2024 US 202418673478**

(71) Applicant: **Siemens Healthineers International AG 6312 Steinhausen (CH)**

(72) Inventors:
• **ROSSI, Michiko**
 **02700 Kaunianen (FI)**
• **LANSONNEUR, Pierre**
 **69004 Lyon (FR)**

(74) Representative: **Mathisen & Macara LLP Charta House 30-38 Church Street Staines-upon-Thames TW18 4EP (GB)**

(54) **DOSE ADJUSTMENT FOR MAXIMUM FIELD DELIVERY TIME IN RADIATION THERAPY TREATMENT PLANNING**

(57)    A proton therapy system 1000 may include at least one processor 102 and a memory 104 storing computer executable instructions. The at least one processor is configured to execute the computer executable instructions to cause the proton therapy system to selectively adjust a treatment plan for proton therapy treatment of a target volume based on a treatment delivery time for the treatment plan and a threshold maximum treatment delivery time, the treatment plan at least prescribing proton therapy field characteristics for treatment of the target volume.

Fig. 1

EP 4 653 046 A1

## Description

## TECHNICAL FIELD

One or more example embodiments relate to radiation therapy treatment planning and a radiation therapy system.

## BACKGROUND

Radiation therapy treatment plan development generally employs medical imaging, such as X-ray, computed tomography (CT), magnetic resonance imaging (MRI), or the like. Typically, a series of two-dimensional patient images, each representing a two-dimensional cross-sectional "slice" of the patient anatomy, are used to reconstruct a three-dimensional representation of a volume of interest (VOI), or structure of interest, from the patient anatomy.

The VOI typically includes one or more organs of interest, often including a planning target volume (PTV), such as a malignant growth or an organ including malignant tissue targeted for radiation therapy; a relatively healthy organ at risk (OAR) in the vicinity of a malignant growth at risk of radiation therapy exposure; or a larger portion of the patient anatomy that includes a combination of one or more PTVs along with one or more OARs. The objective of the radiation therapy treatment plan development is typically to irradiate as much of the PTV as near the prescribed dose as possible, while attempting to minimize irradiation of nearby OARs.

The resulting radiation therapy treatment plans are used during radiation therapy to selectively expose precise areas of the body, such as malignant tumors, to specific doses of radiation in order to destroy the undesirable tissues. During the development of a patient-specific radiation therapy treatment plan, information is generally extracted from the three-dimensional model to determine parameters such as the shape, volume, location, and orientation of one or more PTVs along with one or more OARs. Example treatment modalities making use of treatment plans include intensity modulated radiation therapy (IMRT) and intensity modulated proton therapy (IMPT).

In IMRT, a photon beam includes a number of beam segments or beamlets. The beam is shaped using multi-leaf collimators (MLCs) either before or while the beam is directed into the treatment target. A maximum energy (e.g., 20 MeV) for the beam is specified and an energy for each of the beamlets is determined as a percentage (100 percent or less) or equivalent fraction of the maximum beam energy. Thus, each of the beamlets can be weighted based on its energy level. By weighting based on the energy per beamlet, each beamlet is in effect also weighted based on its intensity.

In IMPT (e.g., spot or pencil beam scanning), a proton or ion beam is directed to spots in a treatment target as prescribed by the treatment plan. The prescribed spot locations are typically arranged in a fixed (raster) pattern for each energy layer of the beam, and the beam is delivered on a fixed scanning path within an energy layer. Each spot may be weighted based on, for example, the number of protons received when irradiated by the beam. The weight of each spot may be expressed as a value of a monitor unit (e.g., number of protons) or MU.

## SUMMARY

The scope of protection sought for various example embodiments is set out by the independent claims. The example embodiments and/or features, if any, described in this specification that do not fall under the scope of the independent claims are to be interpreted as examples useful for understanding various embodiments.

Independent of the grammatical term usage, individuals with male, female or other gender identities are included within the term.

At least one example embodiment provides a proton therapy system comprising: a memory storing computer executable instructions; and at least one processor configured to execute the computer executable instructions to cause the proton therapy system to selectively adjust a treatment plan for proton therapy treatment of a target volume based on a treatment delivery time for the treatment plan and a threshold maximum treatment delivery time, the treatment plan at least prescribing proton therapy field characteristics for treatment of the target volume.

At least one example embodiment provides a proton therapy system comprising: means for selectively adjusting a treatment plan for proton therapy treatment of a target volume based on a treatment delivery time for the treatment plan and a threshold maximum treatment delivery time, the treatment plan at least prescribing proton therapy field characteristics for treatment of the target volume; and means for treating the target volume according to the treatment plan.

At least one example embodiment provides a method comprising: selectively adjusting a treatment plan for proton therapy treatment of a target volume based on a treatment delivery time for the treatment plan and a threshold maximum treatment delivery time, the treatment plan at least prescribing proton therapy field characteristics for treatment of the target volume.

At least one other example embodiment provides a non-transitory computer-readable medium storing computer-executable instructions that, when executed by at least one processor at a proton therapy system, cause the proton therapy system to perform a method comprising: selectively adjusting a treatment plan for proton therapy treatment of a target volume based on a treatment delivery time for the treatment plan and a threshold maximum treatment delivery time, the treatment plan at least prescribing proton therapy field characteristics for treatment of the target volume.

According to one or more example embodiments, selective adjustment of the treatment plan may include selectively adjusting at least one proton therapy field characteristic among the proton therapy field characteristics for the treatment of the target volume.

The at least one proton therapy field characteristic may include at least one of a number of energy layers or a spot spacing for the treatment of the target volume.

The at least one proton therapy field characteristic may include at least one of a number of energy layers or a spot spacing for the treatment of the target volume, and the at least one proton therapy field characteristic may be selectively adjusted by at least one of (i) removing an energy layer from the number of energy layers or (ii) increasing the spot spacing.

The at least one processor may be configured to execute the computer executable instructions to cause the proton therapy system to: determine that the treatment delivery time is greater than the threshold maximum treatment delivery time, and adjust the treatment plan in response to determining that the treatment delivery time is greater than the threshold maximum treatment delivery time.

The at least one processor may be configured to execute the computer executable instructions to cause the proton therapy system to adjust the treatment plan by: adjusting at least one proton therapy field characteristic among the proton therapy field characteristics for the treatment of the target volume, and generating an adjusted treatment plan based on the adjusted at least one proton therapy field characteristic.

The at least one proton therapy field characteristic may include at least one of a number of energy layers or a spot spacing for the treatment of the target volume.

The adjusting at least one proton therapy field characteristic may include at least one of (i) removing an energy layer from the number of energy layers or (ii) increasing the spot spacing.

The proton therapy field characteristics may include at least a number of energy layers and a spot spacing for the treatment of the target volume. The at least one processor may be configured to execute the computer executable instructions to cause the proton therapy system to: determine that the treatment delivery time is greater than the threshold maximum treatment delivery time, adjust at least one of the number of energy layers or the spot spacing in response to determining that the treatment delivery time is greater than the threshold maximum treatment delivery time, and generate an adjusted treatment plan based on the adjusted at least one of the number of energy layers or the spot spacing.

The at least one processor may be configured to execute the computer executable instructions to cause the proton therapy system to adjust at least one of the number of energy layers or the spot spacing by at least one of (i) removing an energy layer from the number of energy layers or (ii) increasing the spot spacing.

The proton therapy field characteristics may include a number of energy layers for the treatment of the target volume. The at least one processor may be configured to execute the computer executable instructions to cause the proton therapy system to: determine that the treatment delivery time is greater than the threshold maximum treatment delivery time, adjust the number of energy layers in response to determining that the treatment delivery time is greater than the threshold maximum treatment delivery time, and generate a first adjusted treatment plan based on the adjusted number of energy layers.

The at least one processor may be configured to execute the computer executable instructions to cause the proton therapy system to adjust the number of energy layers by removing an energy layer from the number of energy layers.

The at least one processor may be configured to execute the computer executable instructions to cause the proton therapy system to: compute a first adjusted treatment delivery time for the first adjusted treatment plan, determine whether the first adjusted treatment delivery time is greater than the threshold maximum treatment delivery time, and output the first adjusted treatment plan for the treatment of the target volume in response to determining that the first adjusted treatment delivery time is less than or equal to the threshold maximum treatment delivery time.

The proton therapy system may further include a radiation therapy machine configured to perform the treatment based on the first adjusted treatment plan.

The proton therapy field characteristics may further include a spot spacing for the treatment of the target volume. The at least one processor may be configured to execute the computer executable instructions to cause the proton therapy system to: determine that the first adjusted treatment delivery time is greater than the threshold maximum treatment delivery time, adjust the spot spacing in response to determining that the first adjusted treatment delivery time is greater than the threshold maximum treatment delivery time, and generate a second adjusted treatment plan based on the adjusted spot spacing.

The proton therapy field characteristics may further include a spot spacing for the treatment of the target volume. The at least one processor may be configured to execute the computer executable instructions to cause the proton therapy system to: compute a first adjusted treatment delivery time for the first adjusted treatment plan, determine that the first adjusted treatment delivery time is greater than the threshold maximum treatment delivery time, adjust the spot spacing in response to determining that the first adjusted treatment delivery time is greater than the threshold maximum treatment delivery time, and generate a second adjusted treatment plan based on the adjusted spot spacing.

The at least one processor may be configured to execute the computer executable instructions to cause the proton therapy system to adjust the spot spacing by increasing the spot spacing.

The at least one processor may be configured to execute the computer executable instructions to cause the proton therapy system to: compute a second adjusted treatment delivery time for the second adjusted treatment plan, determine whether the second adjusted treatment delivery time is greater than the threshold maximum treatment delivery time, and selectively output the second adjusted treatment plan for the treatment of the target volume based on whether the second adjusted treatment delivery time is greater than the threshold maximum treatment delivery time.

The at least one processor may be configured to execute the computer executable instructions to cause the proton therapy system to: output the second adjusted treatment plan for the treatment of the target volume in response to determining that the second adjusted treatment delivery time is less than or equal to the threshold maximum treatment delivery time.

The proton therapy system may further comprise a radiation therapy machine configured to perform the treatment based on the second adjusted treatment plan.

## BRIEF DESCRIPTION OF THE DRAWINGS

Example embodiments will become more fully understood from the detailed description given herein below and the accompanying drawings, wherein like elements are represented by like reference numerals, which are given by way of illustration only and thus are not limiting of this disclosure.

FIG. 1 is a block diagram of an example of a system upon which example embodiments may be implemented.
FIG. 2 illustrates an example of a beam's eye view of a treatment target according to example embodiments.
FIGS. 3 and 4 are block diagrams illustrating examples of an automated radiation therapy treatment planning process according to example embodiments.
FIG. 5 is a flow chart illustrating a method according to example embodiments.
FIG. 6 is a flow chart illustrating a method according to example embodiments.

It should be noted that these figures are intended to illustrate the general characteristics of methods, structure and/or materials utilized in certain example embodiments and to supplement the written description provided below. These drawings are not, however, to scale and may not precisely reflect the precise structural or performance characteristics of any given embodiment and should not be interpreted as defining or limiting the range of values or properties encompassed by example embodiments. The use of similar or identical reference numbers in the various drawings is intended to indicate the presence of a similar or identical element or feature.

## DETAILED DESCRIPTION

Various example embodiments will now be described more fully with reference to the accompanying drawings in which some example embodiments are shown.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

It should be understood that there is no intent to limit example embodiments to the particular forms disclosed. On the contrary, example embodiments are to cover all modifications, equivalents, and alternatives falling within the scope of this disclosure. Like numbers refer to like elements throughout the description of the figures.

As discussed herein the terminology "one or more" and "at least one" may be used interchangeably.

As discussed herein, a radiation therapy treatment plan may also be referred to as a radiation treatment plan, a treatment plan or a plan. Moreover, the terms "proposed" and "candidate" may be used interchangeably in the context of a radiation therapy treatment plan.

It will be appreciated that a number of example embodiments may be used in combination.

Unless specifically stated otherwise as apparent from the following discussion, it is appreciated that throughout the present disclosure, discussions utilizing terms such as "accessing," "determining," "storing," "assigning," "adjusting," "combining," "summing," "adding," "optimizing," "minimizing," producing," "generating," "identifying," "setting," "increasing," "evaluating," "calculating," or the like, may refer to actions and processes of a computer system or similar electronic computing device or processor. The computer system or similar electronic computing device manipulates and transforms data represented as physical (electronic) quantities within the computer system memories, registers or other such information storage, transmission or display devices.

The discussion to follow may include terms such as "weight," "metric," "intensity," "monitor unit," etc. Unless otherwise noted, a value is associated with each such term. For example, a weight (e.g., a weight of a spot or beamlet) has a value, and a metric has a value. For simplicity, the term "weight" or "metric" or "intensity" or "monitor unit" may refer to a value of the weight or metric or intensity or MU itself, unless otherwise noted or apparent from the discussion.

While the operations in one or more flowcharts are presented as occurring in series and in a certain order, example embodiments are not so limited. The operations may be performed in a different order and/or in parallel, and they may also be performed in an iterative manner.

In IMPT (spot or pencil beam scanning), a field includes multiple energy layers, each of which includes a plurality of spots. The spots correspond to successive positions to be irradiated by a pencil beam during treatment and are typically arranged in a fixed (raster) pattern for each energy layer of the beam. Each energy layer corresponds to a depth reached by the pencil beam through the patient. The layer energy and number of energy layers in the field may be determined based on patient geometry and hardware constraints in any known manner.

To irradiate a target volume (also referred to as a treatment target, treatment target volume, or planned target volume (PTV)) during radiation therapy treatment, the target is sliced into the multiple energy layers, and the pencil beam is scanned (e.g., in a fixed scanning path) through the different spot positions of the most distal energy layer in the field direction (direction of the beam). Once all spots of the most distal energy layer are delivered, the pencil beam switches to scan through the different spot positions in the next most distal energy layer in the field direction. The process continues until the pencil beam has scanned through spot positions of all energy layers in the most proximal layer in the field direction, thereby treating the treatment target.

The time required to irradiate a field is referred to as the treatment field delivery time. The treatment field delivery time may also be referred to as the treatment plan delivery time.

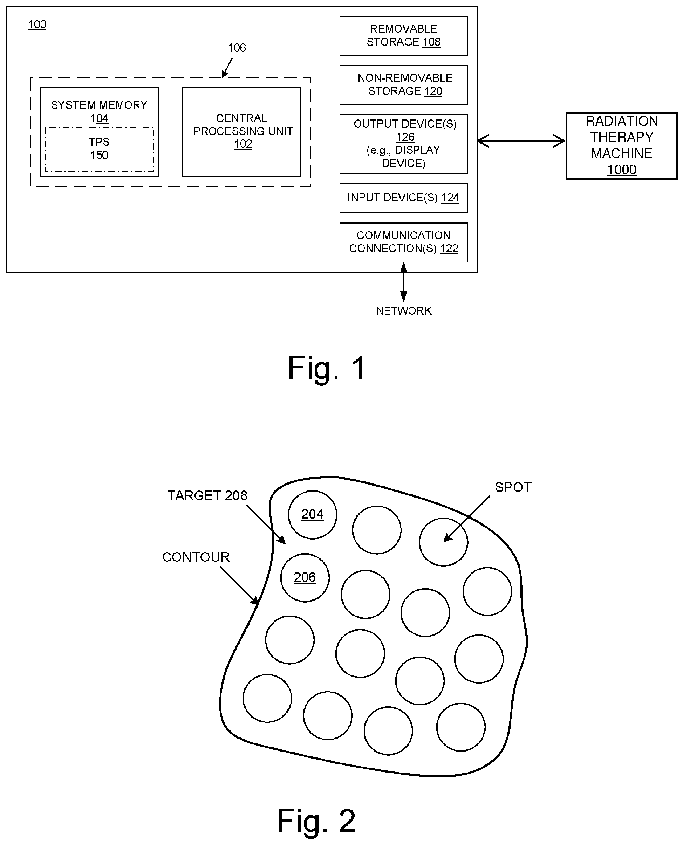

FIG. 2 illustrates an example of a beam's eye view of a treatment target 208 in an IMPT example embodiment. The treatment target 208 may coincide with the shape of the volume being treated (e.g., the contour of the treatment target may coincide with the contour of a tumor), the treatment target may be larger than the volume being treated, or the treatment target may correspond to a portion (e.g., a sub-volume) of the volume being treated.

As shown, an arrangement of spots (e.g., spots 204 and 206) is mapped onto the treatment target 208. Each spot corresponds to a particular location in the treatment target 208. The spots in the treatment target 208 may be irradiated with a raster scan (two-dimensional emission) of a pencil beam as discussed above. As is generally known, each pencil beam can deliver a relatively high dose rate (a relatively high dose in a relatively short period of time) to each spot. For example, if necessary, the pencil beam can deliver above 40 grays (Gy) to each spot in less than one second.

Before a patient is treated with radiation, a treatment plan specific for that patient is developed. The treatment plan defines various aspects of the therapy using simulations and optimizations based on past experiences.

In intensity modulated therapy, the goal of the planner is to find a solution that is optimal with respect to multiple clinical goals that may be contradictory in the sense that an improvement toward one goal may have a detrimental effect on reaching another goal. For example, a treatment plan that spares the liver from receiving a dose of radiation may result in the stomach receiving too much radiation. These types of tradeoffs lead to an iterative process in which the planner creates different plans to find the one plan that is best suited to achieving the desired outcome. Furthermore, treatment planning software can be used to find an optimal plan that considers all the clinical goals and dosimetric criteria.

When generating a treatment plan associated with IMPT, for example, an initial set of spot positions or grid is specified for the entire treatment target, and the plan is optimized by adjusting the weights (number of protons or Monitor Unit (MU)) of the spots in the pattern. An example method for determining a set of spot positions and spot density is described in U.S. Patent Application Publication No. 2023/0405358 to Pierre Lansonneur, the entire contents of which are incorporated herein by reference. Typically, the number of spots in the initial set of spot positions is maintained as low as possible (at a minimum) to reduce the time it takes to optimize the plan and to achieve a high-quality plan with respect to dosimetry. Also, if the initial set of spot positions includes a relatively large number of spots, then the final treatment plan may also include many spots, thus lengthening the treatment time (dose delivery time) to the detriment of the patient.

An advantage of proton therapy over other conventional therapies such as X-ray or neutron radiation therapies is that proton radiation can be limited by depth, and therefore the exposure to inadvertent radiation can be avoided or at least limited by non-target cells having a depth beyond a target calculated area.

By superposition of several proton beams of different energies, a Bragg peak can be spread out to cover target volumes using a uniform, prescribed dose. This enables proton radiation applications to more precisely localize the radiation dosage relative to other types of external beam radiotherapy. During proton therapy treatment, a particle accelerator such as a cyclotron or synchrotron is used to generate a beam of protons from, for example, an internal ion source located in the center of the particle accelerator. The protons in the beam are accelerated (via a generated electric field), and the beam of accelerated protons is subsequently "extracted" and magnetically directed through a series of interconnecting tubes (called a beamline), often through multiple chambers, rooms, or even floors of a building, before finally being applied through a radiation application device at an end section of beam line (often through a radiation nozzle) to a target volume in a treatment room.

A treatment planner may need to ensure that a field is deliverable in less than a specified threshold delivery time due to, for example, a patient not being able to hold his/her breath for a certain period of time.

However, conventional treatment planning systems do not include maximum field delivery time as a requirement in the treatment plan.

One or more example embodiments provide mechanisms to take into account treatment field delivery time in generating a radiation therapy treatment plan. More specifically, for example, one or more example embodiments provide mechanisms for optimizing a radiation therapy treatment plan allowing for treatment field delivery time to be taken into account.

FIG. 1 shows a block diagram of an example of a system upon which one or more example embodiments may be implemented. The system includes a computer system 100 and a radiation therapy machine 1000 in two-way communication with one another.

The radiation therapy machine 1000 may be a treatment modality for providing radiation therapy treatment, such as IMPT.

The computer system 100 includes at least one processing unit 102, memory 104, removable storage 108, non-removable storage 120, and communications connection(s) 122 to enable the system to communicate with other devices (e.g., in a networked environment using logical connections to one or more remote computers).

The system 100 further includes one or more input devices 124 (e.g., a keyboard, mouse, pen, voice input device, touch screen or other input device, etc.) and one or more output devices 126, such as a display device, speakers, printer, etc. The display device may be, for example, a cathode ray tube display, a light-emitting diode display, a liquid crystal display, a touch screen display, a combination thereof, or the like.

In FIG. 1, the memory 104 includes computer-readable instructions, data structures, program modules, and the like associated with a treatment planning system (TPS) 150, which may also be referred to as an optimizer. However, the TPS 150 may instead reside in any one of the computer storage media used by the system 100, or may be distributed over some combination of the computer storage media, or may be distributed over some combination of networked computers. The TPS 150 is configured to generate, optimize and/or evaluate candidate (proposed) treatment plans (e.g., for IMPT) and produce a final (optimized) treatment plan. The final treatment plan may be utilized to apply, via the radiation therapy machine 1000, radiation therapy treatment to a treatment target of a patient.

More specifically, for example, the TPS 150 may define a proposed radiation treatment plan, which is stored in a computer system memory and accessed from that memory. As mentioned above, treatment modalities for the treatment plans may include IMPT.

A radiation therapy treatment plan includes values of parameters that can affect dose and/or dose rate, as well as other parameters. Depending on the treatment modality, the parameters may include, but are not limited to: number and spacing of energy layers in a field, number, arrangement, spacing and density of spots for pencil beam (spot) scanning, and spot weights in each energy layer; beamlet weights; beamlet intensities or energies; beam/beamlet directions; prescribed dose and prescribed dose rate; a number of irradiations of a target volume; a duration of each of the irradiations (irradiation times); and a dose deposited in each of the irradiations. The parameters may also include a period of time during which the irradiations are applied (e.g., a number of irradiations are applied over a period of time such as an hour, with each irradiation in the period of time separated from the next by another period of time) and an interval of time between each period of irradiations (e.g., each hour-long period is separated from the next by a day).

The large number of parameters and their ranges of values can lead to an effectively infinite number of potential treatment plans. Therefore, consistently and efficiently generating and evaluating high-quality treatment plans is beyond the capability of a human and relies on and/or requires the use of a computing system.

To deliver the prescribed dose/dose rate of radiation, the radiation treatment plan may be converted (e.g., by the TPS 150) into machine parameters for a radiation therapy machine (e.g., radiation therapy machine 1000). Machine parameters can include, for example, beam currents of a proton, ion, or photon beam, the number of protons, ions, or photons per time segment to be emitted by the accelerator, magnet currents, settings to achieve the prescribed energy of protons, ions, or photons at the target volume, and the measurement range of a dose monitor system. This conversion thus takes into account the limitations of the treatment machine's equipment that produces the beam and that delivers and monitors the radiation treatment.

During treatment, a beam enters a nozzle of the radiation therapy machine 1000, which may include one or more components that affect (e.g., decrease, modulate, etc.) the energy of the beam, to control the dose/-dose rate delivered by the beam and/or to control the dose versus depth curve of the beam, depending on the type of beam. For example, for a proton beam that has a Bragg peak, the nozzle can control the location of the Bragg peak in the treatment target laterally to the beam axis. In other examples, energy modulation is performed outside of the nozzle (e.g., upstream of the nozzle).

The nozzle is mounted on a moveable gantry so that the beam may be delivered from different directions (angles) relative to a patient (or treatment target of the patient) on the patient support device, and the position of the patient support device relative to the beam may also be changed.

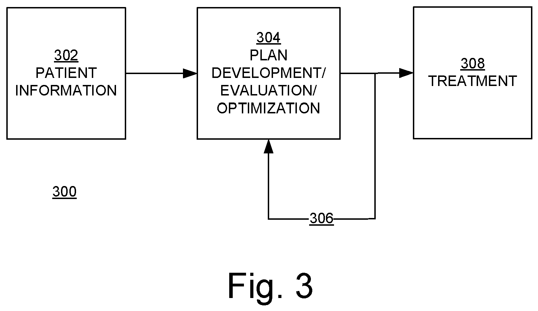

FIG. 3 is a block diagram illustrating an example of an automated radiation therapy treatment planning process or method 300, according to example embodiments. The process 300, in whole or in part, may be implemented as a software program, hardware logic, or a combination thereof on/using the computer system 100 in FIG. 1.

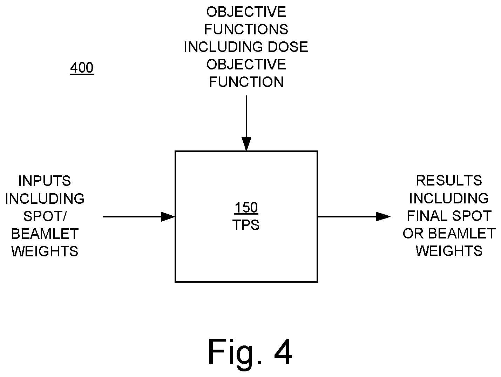

Referring to FIG. 3, in step 302 the computer system 100 obtains three-dimensional (3D) images of a patient, and segments and contours organs and other structures in the patient (the patient geometry). In steps 304 and 306, as will be discussed in more detail below with regard to FIG. 4, the system 100 develops and evaluates a radiation therapy treatment plan based on the information obtained in step 302, and other information.

In step 308, if the treatment plan developed by the system 100 is satisfactory (e.g., satisfies clinical goals), then the radiation therapy treatment plan may be used for treatment of the treatment target of the patient (e.g., using the radiation therapy machine 1000). If not, then the system 100 may iteratively modify aspects of the treatment plan and/or of the clinical goals until a satisfactory plan is generated, and then utilize the satisfactory radiation therapy treatment plan for treatment of the patient (e.g., using the radiation therapy machine 1000). The clinical goals may be expressed in terms of, for example, a set of quality metrics, such as target homogeneity, conformity to the treatment target, critical organ sparing, field delivery time, and the like, with respective target values for the quality metrics.

FIG. 4 is a block diagram illustrating an example of an automated radiation therapy treatment planning process 400, according to example embodiments. The process 400, in whole or in part, may be implemented as a software program, hardware logic, or a combination thereof on/using the system 100 of FIG. 1. For example, the process 400 may be performed at the TPS 150. The process 400 corresponds generally to steps 304 and 306 in FIG. 3.

Referring to FIG. 4, the TPS 150 receives or accesses (e.g., from the memory 104 of FIG. 1) information that includes parameters such as those mentioned above. The TPS 150 may also access or receive information specific to the patient to be treated (e.g., patient geometry), including information that describes a treatment target (region of interest (ROI)), which may include a planned target volume (PTV), gross tumor volume (GTV), clinical target volume (CTV), and/or organs-at-risk (OARs).

The TPS 150 also accesses or receives objective functions defined for the treatment of the patient. Objective functions are mathematical formulations of variables (parameters such as those mentioned above) that may have an effect on achieving specified clinical goals. More specifically, the objective functions are used to evaluate proposed radiation therapy treatment plans to determine whether or not the clinical goals that are specified for treatment of a patient are satisfied.

An example of a dose objective function f(d) is: $f(d) = \Sigma(w_i)(d_i - d_p)^2$, where $w_i$ is a weight per voxel in a treatment target, $d_i$ is the dose per voxel projected to be received according to a proposed treatment plan, $d_p$ is the prescribed dose per voxel, and the summation $\Sigma$ is

over all voxels i in the treatment target. As noted above in IMPT, a voxel may be a spot in the treatment target irradiated by the pencil beam. In this example, the goal is to minimize the value of the dose objective function (in this case, the dose across the treatment target becomes more uniform as the value of the function decreases). In practice, there may be several objective functions (in addition to the dose objective function) that are to be minimized in order to achieve an optimal final treatment plan. The objective functions may conflict with each other; that is, minimizing one objective function may penalize another objective function, and so minimizing all of the objective functions may not be achievable. Thus, in example embodiments, the objective functions may be weighted and summed to provide a total of all of the objective functions, and that total may then be minimized.

As noted above, a treatment planner may need to ensure that a field is deliverable in less than a specified threshold delivery time due to, for example, a patient not being able to hold his/her breath for a certain period of time. However, conventional treatment planning systems do not include maximum field delivery time as a requirement in the treatment plan.

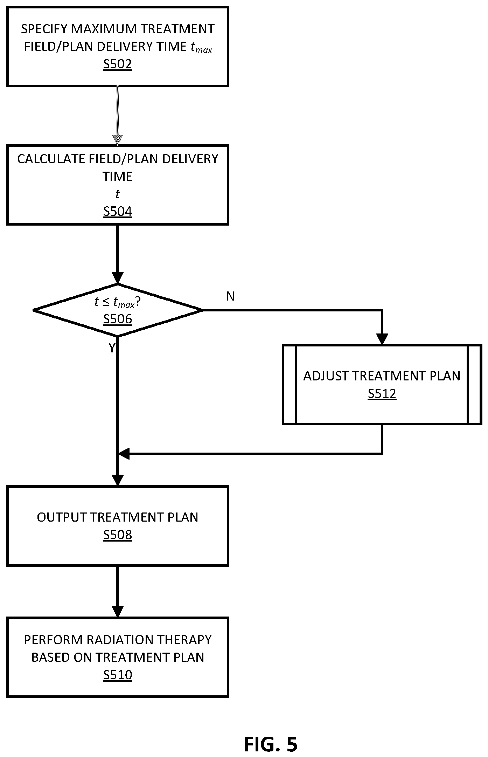

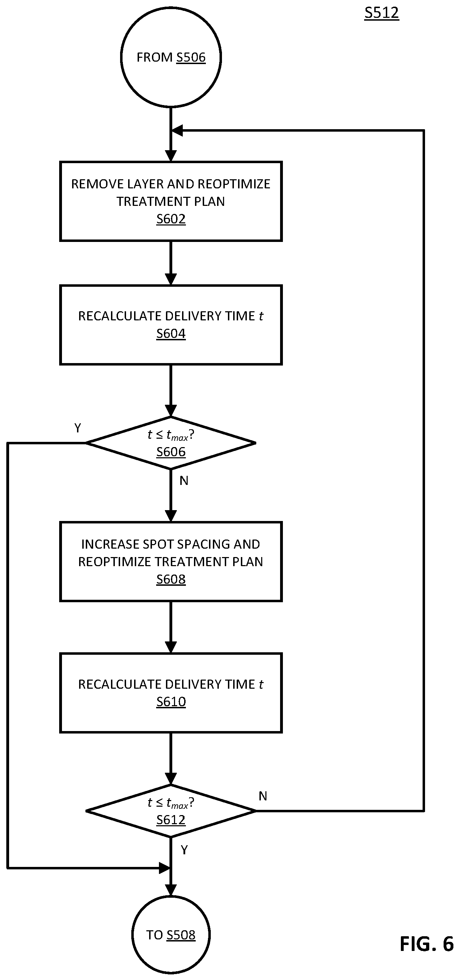

At least the example embodiments shown in FIGS. 5 and 6 provide mechanisms to take into account treatment field delivery time in generating and/or adjusting a radiation therapy treatment plan. More specifically, for example, one or more example embodiments provide mechanisms for optimizing a radiation therapy treatment plan allowing for treatment field delivery time to be taken into account.

FIG. 5 is a flow chart illustrating a method for radiation therapy treatment planning and treatment, according to example embodiments. The method shown in FIG. 5 will be discussed as being performed by the system shown in FIG. 1. Example embodiments should not, however, be limited to this example.

In accordance with the method shown in FIG. 5, the system shown in FIG. 1 (e.g., the TPS 150) may selectively adjust a treatment plan for proton therapy treatment (e.g., IMPT) of a target volume based on a treatment field delivery time (also referred to herein as a treatment plan delivery time) for the treatment plan and a threshold maximum treatment delivery time. The treatment plan at least prescribes proton therapy field characteristics for treatment of the target volume. The system shown in FIG. 1 may then perform radiation therapy treatment based on the selectively adjusted treatment plan.

Referring to FIG. 5, at S502 the TPS 150 specifies a threshold maximum treatment field delivery time $t_{max}$ for delivery of the radiation therapy treatment to the target volume. The maximum treatment field delivery time $t_{max}$ may be specified by a treatment planning user. In at least one example, the maximum treatment field delivery time $t_{max}$ may be equal to the length of one breath-hold by a patient. For example, the maximum treatment field delivery time $t_{max}$ may be less than or equal to about 20 seconds.

At S504, the TPS 150 calculates an initial treatment field delivery time $t$ for an identified (e.g., proposed or candidate) radiation therapy treatment plan. The identified radiation therapy treatment plan may be obtained from memory and/or generated as described above with regard to FIGS. 3 and 4. The initial treatment field delivery time $t$ may be computed as the sum of the delivery time per energy for the energy layers in the field. The delivery time for a given energy layer is based on the position and MU values of the spots and the machine parameters of the radiation therapy machine delivering the radiation therapy. For example, the delivery time for a given energy layer may be the sum of the time required to deliver the required dose to each spot and the time required to move between spots included in the energy layer. Because methods for computing delivery time are generally known, a further discussion is omitted for the sake of brevity.

At S506, the TPS 150 determines whether the treatment field delivery time $t$ (also referred to as a treatment delivery time or treatment plan delivery time) is less than or equal to the maximum treatment field delivery time $t_{max}$ (also referred to herein as a threshold maximum treatment delivery time). That is, for example, the TPS 150 determines whether the treatment field delivery time satisfies (is within) a maximum treatment field delivery time constraint by comparing the treatment field delivery time $t$ with the maximum treatment field delivery time $t_{max}$.

If the treatment field delivery time $t$ is less than or equal to the maximum treatment field delivery time $t_{max}$, then the TPS 150 may determine that the plan is acceptable, at least with regard to the treatment field delivery time. At S508, if the treatment plan is also satisfactory as discussed above with regard to FIG. 3, the TPS 150 may output the treatment plan to the radiation therapy machine 1000 for treatment.

At S510, the radiation therapy machine 1000 may perform radiation therapy treatment on the target volume according to the radiation treatment plan from the TPS 150.

Returning to S506, if the treatment field delivery time $t$ is greater than the maximum treatment field delivery time $t_{max}$, then at S512 the TPS 150 adjusts the radiation therapy treatment plan based on the treatment field delivery time $t$ and the maximum treatment field delivery time $t_{max}$ such that the adjusted treatment plan at least satisfies the maximum treatment field delivery time constraint (treatment field delivery time $t$ is less than or equal to the threshold maximum treatment field delivery time constraint ($t \leq t_{max}$)) and is otherwise satisfactory. In at least one example embodiment, the TPS 150 may adjust the radiation therapy treatment plan by adjusting (e.g., iteratively adjusting) at least one proton therapy field characteristic among the proton therapy field characteristics for the treatment of the target volume and reoptimize the treatment plan, until the treatment field delivery time $t$ satisfies the threshold maximum treatment field delivery time constraint ($t \leq t_{max}$). The at least one proton therapy field characteristic may include at least one of a number of energy layers or a spot spacing for the treatment of the target volume. The at least one proton therapy field characteristic may be adjusted by at least one of (i) removing an energy layer from the number of energy layers in the field or (ii) increasing the spot spacing within one or more energy layers of the field.

An example embodiment of a method for adjusting the radiation therapy treatment plan at S512 will be discussed in more detail below with regard to FIG. 6.

Still referring to FIG. 5, once having obtained a radiation therapy treatment plan satisfying the maximum treatment field delivery time constraint and that is otherwise satisfactory, the process proceeds to S508 and continues as discussed above.

FIG. 6 is a flow chart illustrating a method for adjusting a radiation therapy treatment plan at S512, according to example embodiments. Similar to FIG. 5, the method shown in FIG. 6 will be discussed as being performed by the TPS 150 shown in FIG. 1. Example embodiments should not, however, be limited to this example.

Referring to FIG. 6, once having determined that the treatment field delivery time t is greater than or equal to the threshold maximum treatment field delivery time constraint ($t > t_{max}$)) at S506, at S602 the TPS 150 removes an energy layer from the radiation therapy treatment field and reoptimizes the adjusted treatment plan based on the remaining energy layers in the field. In one example, the remaining energy layers may be redistributed and the treatment plan optimized in any known manner.

At S604, the TPS 150 recalculates the treatment field delivery time t for the adjusted radiation therapy treatment plan determined at S602. The TPS 150 calculates the treatment field delivery time $t$ in the same or substantially the same manner as discussed above with regard to S504 in FIG. 5.

At S606, the TPS 150 again determines whether the treatment field delivery time $t$ for the adjusted treatment plan satisfies the maximum treatment field delivery time constraint.

If the TPS 150 determines that the treatment field delivery time $t$ for the adjusted treatment plan satisfies the maximum treatment field delivery time constraint at S606, then the process proceeds to S508 and continues as discussed above with regard to FIG. 5.

Returning to S606, if the TPS 150 determines that the treatment field delivery time $t$ for the adjusted treatment plan does not satisfy the maximum treatment field delivery time constraint, then at S608 the TPS 150 further adjusts the radiation therapy treatment plan by adjusting the spot spacing for the treatment field. In one example, the TPS 150 adjusts the spot spacing by increasing the spot spacing for energy layers of the treatment field. The spot spacing may be increased at a fixed distance for the energy layers in the field or maybe adjusted layer-by-layer based on, for example, spot size. Also at S608, the TPS 150 reoptimizes the further adjusted treatment plan with the increased spot spacing.

In at least one example embodiment, the TPS 150 may compute an adjusted spot positioning (including spacing) based on the minimum MU per spot in a field constraint. In one example, the TPS 150 may compute the distance (spacing) $s$ between spots in the field to satisfy the minimum MU constraint according to Equation (1) shown below, where $MU_{min}$ is the minimum MU per spot in the field, $MU_{field}$ is the total MU for the field, and $A$ is the total covered area of the target volume. To compute the adjusted spot spacing, the TPS 150 may adjust one or more of the parameters in Equation (1) as needed.

$$s_{min} = \sqrt{\frac{A \cdot MU_{min}}{MU_{field}}} \text{he}$$

he minimum distance between spots $s_{min}$ in Equation (1) is directly proportional to the minimum MU per spot constraint, the larger the minimum MU per spot, the wider apart the spots will need to be placed when determining the set of spot positions. Spots may be evenly spaced by a distance on a regular grid to cover a cross section of the respective PTV as seen from the beam eye view. Accordingly, the spot spacing s for a target volume where the minimum MU per spot is 400 is higher than the spacing s' for the target volume where the minimum MU per spot is 100.

According to one or more example embodiments, the information regarding the spot spacing or density of spots based on the minimum Monitor Unit (MU) criteria may be combined with certain optimization objectives, for example, for PTVs and OARs to derive a set of spot positions (e.g., using a density map and weighted Voronoi stippling process (a rendering process)) that fulfill both the minimum MU constraint and the optimization objectives. Further discussion of determining spot spacing may be found in U.S. Patent Application Publication No. 2023/0405358 to Pierre Lansonneur, the entire contents of which are incorporated herein by reference. However, example embodiments should not be limited to this example. Rather, other methodologies for determining and/or adjusting spot spacing may also be used.

At S610, the TPS 150 again recalculates the treatment field delivery time t for the further adjusted radiation therapy treatment plan determined at S608. The TPS 150 calculates the treatment field delivery time $t$ in the same or substantially the same manner as discussed above with regard to S504 in FIG. 5.

At S612, the TPS 150 again determines whether the treatment field delivery time $t$ for the further adjusted treatment plan satisfies the maximum treatment field delivery time constraint.

If the TPS 150 determines that the treatment field delivery time $t$ for the further adjusted treatment plan satisfies the maximum treatment field delivery time constraint at S612, then the process proceeds to S508 and continues as discussed above.

Returning to S612, however, if the TPS 150 determines that the treatment field delivery time $t$ for the further adjusted treatment plan does not satisfy the maximum treatment field delivery time constraint at S612, then the process returns to S602 and continues as discussed herein to iteratively adjust the radiation therapy treatment plan until the maximum treatment field delivery time constraint is satisfied.

By way of one or more example embodiments, a treatment planner may ensure that a treatment field is deliverable in less than a threshold time period as needed due to, for example, a patient not being able to hold their breath for a certain period of time.

Additionally, the treatment planner need not use a brute-force method of optimizing the dose and taking the field delivery time into account by iterative searching.

Illustrative embodiment 1. A proton therapy system comprising: a memory storing computer executable instructions; and at least one processor configured to execute the computer executable instructions to cause the proton therapy system to selectively adjust a treatment plan for proton therapy treatment of a target volume based on a treatment delivery time for the treatment plan and a threshold maximum treatment delivery time, the treatment plan at least prescribing proton therapy field characteristics for treatment of the target volume.

Illustrative embodiment 2. The proton therapy system of illustrative embodiment 1, wherein selective adjustment of the treatment plan includes selectively adjusting at least one proton therapy field characteristic among the proton therapy field characteristics for the treatment of the target volume.

Illustrative embodiment 3. The proton therapy system of illustrative embodiment 2, wherein the at least one proton therapy field characteristic includes at least one of a number of energy layers or a spot spacing for the treatment of the target volume.

Illustrative embodiment 4. The proton therapy system of any of illustrative embodiments 2 or 3, wherein the at least one proton therapy field characteristic includes at least one of a number of energy layers or a spot spacing for the treatment of the target volume, and the at least one proton therapy field characteristic is selectively adjusted by at least one of (i) removing an energy layer from the number of energy layers or (ii) increasing the spot spacing.

Illustrative embodiment 5. The proton therapy system of any of illustrative embodiments 1-4, wherein the at least one processor is configured to execute the computer executable instructions to cause the proton therapy system to: determine that the treatment delivery time is greater than the threshold maximum treatment delivery time, and adjust the treatment plan in response to determining that the treatment delivery time is greater than the threshold maximum treatment delivery time.

Illustrative embodiment 6. The proton therapy system of illustrative embodiment 5, wherein the at least one processor is configured to execute the computer executable instructions to cause the proton therapy system to adjust the treatment plan by: adjusting at least one proton therapy field characteristic among the proton therapy field characteristics for the treatment of the target volume, and generating an adjusted treatment plan based on the adjusted at least one proton therapy field characteristic.

Illustrative embodiment 7. The proton therapy system of illustrative embodiment 6, wherein the at least one proton therapy field characteristic includes at least one of a number of energy layers or a spot spacing for the treatment of the target volume.

Illustrative embodiment 8. The proton therapy system of illustrative embodiment 7, wherein the adjusting at least one proton therapy field characteristic includes at least one of (i) removing an energy layer from the number of energy layers or (ii) increasing the spot spacing.

Illustrative embodiment 9. The proton therapy system of any of illustrative embodiments 1-8, wherein the proton therapy field characteristics include at least a number of energy layers and a spot spacing for the treatment of the target volume, and the at least one processor is configured to execute the computer executable instructions to cause the proton therapy system to: determine that the treatment delivery time is greater than the threshold maximum treatment delivery time, adjust at least one of the number of energy layers or the spot spacing in response to determining that the treatment delivery time is greater than the threshold maximum treatment delivery time, and generate an adjusted treatment plan based on the adjusted at least one of the number of energy layers or the spot spacing.

Illustrative embodiment 10. The proton therapy system of illustrative embodiment 9, wherein the at least one processor is configured to execute the computer executable instructions to cause the proton therapy system to adjust at least one of the number of energy layers or the spot spacing by at least one of (i) removing an energy layer from the number of energy layers or (ii) increasing the spot spacing.

Illustrative embodiment 11. The proton therapy system of any of illustrative embodiments 1-10, wherein the proton therapy field characteristics include a number of energy layers for the treatment of the target volume, and the at least one processor is configured to execute the computer executable instructions to cause the proton therapy system to: determine that the treatment delivery time is greater than the threshold maximum treatment delivery time, adjust the number of energy layers in response to determining that the treatment delivery time is greater than the threshold maximum treatment delivery time, and generate a first adjusted treatment plan based on the adjusted number of energy layers.

Illustrative embodiment 12. The proton therapy system of illustrative embodiment 11, wherein the at least one processor is configured to execute the computer executable instructions to cause the proton therapy system to adjust the number of energy layers by removing an energy layer from the number of energy layers.

Illustrative embodiment 13. The proton therapy system of any of illustrative embodiments 11-12, wherein the at least one processor is configured to execute the computer executable instructions to cause the proton therapy system to: compute a first adjusted treatment delivery time for the first adjusted treatment plan, determine whether the first adjusted treatment delivery time is greater than the threshold maximum treatment delivery time, and output the first adjusted treatment plan for the treatment of the target volume in response to determining that the first adjusted treatment delivery time is less than or equal to the threshold maximum treatment delivery time.

Illustrative embodiment 14. The proton therapy system of illustrative embodiment 13, further comprising: a radiation therapy machine configured to perform the treatment based on the first adjusted treatment plan.

Illustrative embodiment 15. The proton therapy system of any of illustrative embodiments 13-14, wherein the proton therapy field characteristics further include a spot spacing for the treatment of the target volume, and the at least one processor is configured to execute the computer executable instructions to cause the proton therapy system to: determine that the first adjusted treatment delivery time is greater than the threshold maximum treatment delivery time, adjust the spot spacing in response to determining that the first adjusted treatment delivery time is greater than the threshold maximum treatment delivery time, and generate a second adjusted treatment plan based on the adjusted spot spacing.

Illustrative embodiment 16. The proton therapy system of any of illustrative embodiments 11-15, wherein the proton therapy field characteristics further include a spot spacing for the treatment of the target volume, and the at least one processor is configured to execute the computer executable instructions to cause the proton therapy system to: compute a first adjusted treatment delivery time for the first adjusted treatment plan, determine that the first adjusted treatment delivery time is greater than the threshold maximum treatment delivery time, adjust the spot spacing in response to determining that the first adjusted treatment delivery time is greater than the threshold maximum treatment delivery time, and generate a second adjusted treatment plan based on the adjusted spot spacing.

Illustrative embodiment 17. The proton therapy system of illustrative embodiment 16, wherein the at least one processor is configured to execute the computer executable instructions to cause the proton therapy system to adjust the spot spacing by increasing the spot spacing.

Illustrative embodiment 18. The proton therapy system of any of illustrative embodiments 16-17, wherein the at least one processor is configured to execute the computer executable instructions to cause the proton therapy system to: compute a second adjusted treatment delivery time for the second adjusted treatment plan, determine whether the second adjusted treatment delivery time is greater than the threshold maximum treatment delivery time, and selectively output the second adjusted treatment plan for the treatment of the target volume based on whether the second adjusted treatment delivery time is greater than the threshold maximum treatment delivery time.

Illustrative embodiment 19. The proton therapy system of illustrative embodiment 18, wherein the at least one processor is configured to execute the computer executable instructions to cause the proton therapy system to: output the second adjusted treatment plan for the treatment of the target volume in response to determining that the second adjusted treatment delivery time is less than or equal to the threshold maximum treatment delivery time.

Illustrative embodiment 20. The proton therapy system of illustrative embodiment 19, further comprising: a radiation therapy machine configured to perform the treatment based on the second adjusted treatment plan.

Although the terms first, second, etc. may be used herein to describe various elements, these elements should not be limited by these terms. These terms are only used to distinguish one element from another. For example, a first element could be termed a second element, and similarly, a second element could be termed a first element, without departing from the scope of this disclosure. As used herein, the term "and/or," includes any and all combinations of one or more of the associated listed items.

When an element is referred to as being "connected," or "coupled," to another element, it can be directly connected or coupled to the other element or intervening elements may be present. By contrast, when an element is referred to as being "directly connected," or "directly coupled," to another element, there are no intervening elements present. Other words used to describe the relationship between elements should be interpreted in a like fashion (e.g., "between," versus "directly between," "adjacent," versus "directly adjacent," etc.).

The terminology used herein is for the purpose of describing particular embodiments only and is not intended to be limiting. As used herein, the singular forms "a," "an," and "the," are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Specific details are provided in the following description to provide a thorough understanding of example embodiments. However, it will be understood by one of ordinary skill in the art that example embodiments may be practiced without these specific details. For example, systems may be shown in block diagrams so as not to obscure the example embodiments in unnecessary detail. In other instances, well-known processes, structures and techniques may be shown without unnecessary detail in order to avoid obscuring example embodiments.

As discussed herein, illustrative embodiments will be described with reference to acts and symbolic representations of operations (e.g., in the form of flow charts, flow diagrams, data flow diagrams, structure diagrams, block diagrams, etc.) that may be implemented as program modules or functional processes include routines, programs, objects, components, data structures, etc., that perform particular tasks or implement particular abstract data types and may be implemented using existing hardware, for example, processing or control circuitry such as, but not limited to, one or more processors, one or more Central Processing Units (CPUs), one or more controllers, one or more arithmetic logic units (ALUs), one or more digital signal processors (DSPs), one or more microcomputers, one or more field programmable gate arrays (FPGAs), one or more System-on-Chips (SoCs), one or more programmable logic units (PLUs), one or more microprocessors, one or more Application Specific Integrated Circuits (ASICs), or any other device or devices capable of responding to and executing instructions in a defined manner.

Although a flow chart may describe the operations as a sequential process, many of the operations may be performed in parallel, concurrently or simultaneously. In addition, the order of the operations may be re-arranged. A process may be terminated when its operations are completed, but may also have additional steps not included in the figure. A process may correspond to a method, function, procedure, subroutine, subprogram, etc. When a process corresponds to a function, its termination may correspond to a return of the function to the calling function or the main function.

As disclosed herein, the term "memory," "storage medium," "processor readable medium," "computer readable storage medium" or "non-transitory computer readable storage medium" may represent one or more devices for storing data, including read only memory (ROM), random access memory (RAM), magnetic RAM, core memory, magnetic disk storage mediums, optical storage mediums, flash memory devices and/or other tangible machine-readable mediums for storing information. The term "computer-readable medium" may include, but is not limited to, portable or fixed storage devices, optical storage devices, and various other mediums capable of storing, containing or carrying instruction(s) and/or data.

Furthermore, example embodiments may be implemented by hardware, software, firmware, middleware, microcode, hardware description languages, or any combination thereof. When implemented in software, firmware, middleware or microcode, the program code or code segments to perform the necessary tasks may be stored in a machine or computer readable medium such as a computer readable storage medium. When implemented in software, a processor or processors will perform the necessary tasks. For example, as mentioned above, according to one or more example embodiments, at least one memory may include or store computer program code, and the at least one memory and the computer program code may be configured to, with at least one processor, cause a network element or network device to perform the necessary tasks. Additionally, the processor, memory and example algorithms, encoded as computer program code, serve as means for providing or causing performance of operations discussed herein.

The terms "including" and/or "having," as used herein, are defined as comprising (i.e., open language). The term "coupled," as used herein, is defined as connected, although not necessarily directly, and not necessarily mechanically. Terminology derived from the word "indicating" (e.g., "indicates" and "indication") is intended to encompass all the various techniques available for communicating or referencing the object/information being indicated. Some, but not all, examples of techniques available for communicating or referencing the object/information being indicated include the conveyance of the object/information being indicated, the conveyance of an identifier of the object/information being indicated, the conveyance of information used to generate the object/information being indicated, the conveyance of some part or portion of the object/information being indicated, the conveyance of some derivation of the object/information being indicated, and the conveyance of some symbol representing the object/information being indicated.

According to example embodiments, medical systems, may be (or include) hardware, firmware, hardware executing software or any combination thereof. Such hardware may include processing or control circuitry such as, but not limited to, one or more processors, one or more CPUs, one or more controllers, one or more ALUs, one or more DSPs, one or more microcomputers, one or more FPGAs, one or more SoCs, one or more PLUs, one or more microprocessors, one or more ASICs, or any other device or devices capable of responding to and executing instructions in a defined manner.

Benefits, other advantages, and solutions to problems have been described above with regard to specific embodiments. However, the benefits, advantages, solutions to problems, and any element(s) that may cause or result in such benefits, advantages, or solutions, or cause such benefits, advantages, or solutions to become more pronounced are not to be construed as a critical, required, or essential feature or element of any or all the claims.

**Claims**

1. A proton therapy system comprising:

   a memory storing computer executable instructions; and
   at least one processor configured to execute the computer executable instructions to cause the proton therapy system to selectively adjust a treatment plan for proton therapy treatment of a target volume based on a treatment delivery time for the treatment plan and a threshold maximum treatment delivery time, the treatment plan at least prescribing proton therapy field characteristics for treatment of the target volume.

2. The proton therapy system of claim 1, wherein selective adjustment of the treatment plan includes selectively adjusting at least one proton therapy field characteristic among the proton therapy field characteristics for the treatment of the target volume.

3. The proton therapy system of claim 2, wherein the at least one proton therapy field characteristic includes at least one of a number of energy layers or a spot spacing for the treatment of the target volume.

4. The proton therapy system of claim 2, wherein

   the at least one proton therapy field characteristic includes at least one of a number of energy layers or a spot spacing for the treatment of the target volume, and
   the at least one proton therapy field characteristic is selectively adjusted by at least one of (i) removing an energy layer from the number of energy layers or (ii) increasing the spot spacing.

5. The proton therapy system of claim 1, wherein the at least one processor is configured to execute the computer executable instructions to cause the proton therapy system to

   determine that the treatment delivery time is greater than the threshold maximum treatment delivery time, and
   adjust the treatment plan in response to determining that the treatment delivery time is greater than the threshold maximum treatment delivery time.

6. The proton therapy system of claim 5, wherein the at least one processor is configured to execute the computer executable instructions to cause the proton therapy system to adjust the treatment plan by

adjusting at least one proton therapy field characteristic among the proton therapy field characteristics for the treatment of the target volume, and

generating an adjusted treatment plan based on the adjusted at least one proton therapy field characteristic.

7. The proton therapy system of claim 6, wherein the at least one proton therapy field characteristic includes at least one of a number of energy layers or a spot spacing for the treatment of the target volume.

8. The proton therapy system of claim 7, wherein the adjusting at least one proton therapy field characteristic includes at least one of (i) removing an energy layer from the number of energy layers or (ii) increasing the spot spacing.

9. The proton therapy system of claim 1, wherein

the proton therapy field characteristics include at least a number of energy layers and a spot spacing for the treatment of the target volume, and

the at least one processor is configured to execute the computer executable instructions to cause the proton therapy system to

determine that the treatment delivery time is greater than the threshold maximum treatment delivery time,

adjust at least one of the number of energy layers or the spot spacing in response to determining that the treatment delivery time is greater than the threshold maximum treatment delivery time, and

generate an adjusted treatment plan based on the adjusted at least one of the number of energy layers or the spot spacing.

10. The proton therapy system of claim 9, wherein the at least one processor is configured to execute the computer executable instructions to cause the proton therapy system to adjust at least one of the number of energy layers or the spot spacing by at least one of (i) removing an energy layer from the number of energy layers or (ii) increasing the spot spacing.

11. The proton therapy system of claim 1, wherein

the proton therapy field characteristics include a number of energy layers for the treatment of the target volume, and

the at least one processor is configured to execute the computer executable instructions to cause the proton therapy system to

determine that the treatment delivery time is greater than the threshold maximum treatment delivery time,

adjust the number of energy layers in response to determining that the treatment delivery time is greater than the threshold maximum treatment delivery time, and

generate a first adjusted treatment plan based on the adjusted number of energy layers.

12. The proton therapy system of claim 11, wherein the at least one processor is configured to execute the computer executable instructions to cause the proton therapy system to adjust the number of energy layers by removing an energy layer from the number of energy layers.

13. The proton therapy system of claim 11 or 12, wherein the at least one processor is configured to execute the computer executable instructions to cause the proton therapy system to

compute a first adjusted treatment delivery time for the first adjusted treatment plan,

determine whether the first adjusted treatment delivery time is greater than the threshold maximum treatment delivery time,

output the first adjusted treatment plan for the treatment of the target volume in response to determining that the first adjusted treatment delivery time is less than or equal to the threshold maximum treatment delivery time.

14. The proton therapy system of claim 13, further comprising:

a radiation therapy machine configured to perform the treatment based on the first adjusted treatment plan.

15. The proton therapy system of claim 13 or 14, wherein

the proton therapy field characteristics further include a spot spacing for the treatment of the target volume, and

the at least one processor is configured to execute the computer executable instructions to cause the proton therapy system to

determine that the first adjusted treatment delivery time is greater than the threshold maximum treatment delivery time,

adjust the spot spacing in response to determining that the first adjusted treatment delivery time is greater than the threshold maximum treatment delivery time, and

generate a second adjusted treatment plan based on the adjusted spot spacing; and,

optionally:
the proton therapy system comprises a radiation therapy machine configured to perform the treatment based on the second adjusted treatment plan;
and/or:

the at least one processor is configured to execute the computer executable instructions to cause the proton therapy system to adjust the spot spacing by increasing the spot spacing, or
the at least one processor is configured to execute the computer executable instructions to cause the proton therapy system to compute a second adjusted treatment delivery time for the second adjusted treatment plan, determine whether the second adjusted treatment delivery time is greater than the threshold maximum treatment delivery time, and selectively output the second adjusted treatment plan for the treatment of the target volume based on whether the second adjusted treatment delivery time is greater than the threshold maximum treatment delivery time, the second adjusted treatment plan for the treatment of the target volume optionally being output in response to determining that the second adjusted treatment delivery time is less than or equal to the threshold maximum treatment delivery time.

Fig. 1

Fig. 2

302
PATIENT
INFORMATION

304
PLAN
DEVELOPMENT/
EVALUATION/
OPTIMIZATION

308
TREATMENT

300

306

Fig. 3

<u>400</u>

OBJECTIVE
FUNCTIONS
INCLUDING DOSE
OBJECTIVE
FUNCTION

INPUTS
INCLUDING
SPOT/
BEAMLET
WEIGHTS

<u>150</u>
TPS

RESULTS
INCLUDING
FINAL SPOT
OR BEAMLET
WEIGHTS

# Fig. 4

SPECIFY MAXIMUM TREATMENT
FIELD/PLAN DELIVERY TIME $t_{max}$
S502

CALCULATE FIELD/PLAN DELIVERY
TIME
$t$
S504

$t \le t_{max}$?
S506

N

Y

ADJUST TREATMENT PLAN
S512

OUTPUT TREATMENT PLAN
S508

PERFORM RADIATION THERAPY
BASED ON TREATMENT PLAN
S510

**FIG. 5**

S512

FROM S506

REMOVE LAYER AND REOPTIMIZE
TREATMENT PLAN
S602

RECALCULATE DELIVERY TIME $t$
S604

$t \leq t_{max}$?
S606

Y

N

INCREASE SPOT SPACING AND
REOPTIMIZE TREATMENT PLAN
S608

RECALCULATE DELIVERY TIME $t$
S610

$t \leq t_{max}$?
S612

N

Y

TO S508

**FIG. 6**

Europäisches Patentamt

European Patent Office

Office européen des brevets

# EUROPEAN SEARCH REPORT

**Application Number**

EP 25 17 8182

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/176156 A1 (MEIJERS ARTURS [US] ET AL) 9 June 2022 (2022-06-09) * paragraphs [0007], [0042], [0050], [0053], [0054], [0067], [0068]; figure 12 * | 1-15 | INV. A61N5/10 |
| X | US 2018/078785 A1 (OLLILA SANTTU [FI] ET AL) 22 March 2018 (2018-03-22) * paragraphs [0030], [0043], [0104], [0106] * | 1,2,5,6 | |
| X | US 2023/271030 A1 (VANDERSTRAETEN REYNALD VANDERSTRAETEN [BE] ET AL) 31 August 2023 (2023-08-31) * paragraphs [0008], [0009], [0013], [0123], [0130], [0131] * | 1,2,5,6 | |
| A | US 2021/379405 A1 (HUTH ISABEL [DE] ET AL) 9 December 2021 (2021-12-09) * paragraphs [0013], [0016], [0064], [0069] - [0075], [0080], [0084] * | 1-15 | |

TECHNICAL FIELDS SEARCHED (IPC)

A61N

The present search report has been drawn up for all claims

| Place of search | Date of completion of the search | Examiner |
|---|---|---|
| Munich | 28 August 2025 | Link, Tatiana |

EPO FORM 1503 03.82 (P04C01)

## ANNEX TO THE EUROPEAN SEARCH REPORT
## ON EUROPEAN PATENT APPLICATION NO.

EP 25 17 8182

This annex lists the patent family members relating to the patent documents cited in the above-mentioned European search report.
The members are as contained in the European Patent Office EDP file on
The European Patent Office is in no way liable for these particulars which are merely given for the purpose of information.

28-08-2025

| Patent document cited in search report | | Publication date | Patent family member(s) | | Publication date |
|---|---|---|---|---|---|
| US 2022176156 | A1 | 09-06-2022 | CN | 114602066 A | 10-06-2022 |
| | | | EP | 4011443 A1 | 15-06-2022 |
| | | | US | 2022176156 A1 | 09-06-2022 |
| US 2018078785 | A1 | 22-03-2018 | EP | 3515555 A1 | 31-07-2019 |
| | | | US | 2018078785 A1 | 22-03-2018 |
| | | | WO | 2018050886 A1 | 22-03-2018 |
| US 2023271030 | A1 | 31-08-2023 | CN | 110709134 A | 17-01-2020 |
| | | | CN | 115054835 A | 16-09-2022 |
| | | | EP | 3655100 A1 | 27-05-2020 |
| | | | US | 10092774 B1 | 09-10-2018 |
| | | | US | 2019060667 A1 | 28-02-2019 |
| | | | US | 2021052917 A1 | 25-02-2021 |
| | | | US | 2023271030 A1 | 31-08-2023 |
| | | | WO | 2019016305 A1 | 24-01-2019 |
| US 2021379405 | A1 | 09-12-2021 | CN | 110960805 A | 07-04-2020 |
| | | | CN | 116271576 A | 23-06-2023 |
| | | | EP | 3636318 A1 | 15-04-2020 |
| | | | EP | 4212209 A1 | 19-07-2023 |
| | | | US | 2020105395 A1 | 02-04-2020 |
| | | | US | 2020406060 A1 | 31-12-2020 |
| | | | US | 2021379405 A1 | 09-12-2021 |

EPO FORM P0459

For more details about this annex : see Official Journal of the European Patent Office, No. 12/82

**EP 4 653 046 A1**

**REFERENCES CITED IN THE DESCRIPTION**

**Patent documents cited in the description**

• US 20230405358, Pierre Lansonneur